# EUROPEAN PATENT SPECIFICATION

(11) **EP 4 088 721 B1**
(45) Date of publication and mention of the grant of the patent: **17.09.2025**
(21) Application number: 21738899.0
(22) Date of filing: 08.01.2021
(51) Int. Cl.: A61K 31/48, A61P 25/00, A23L 33/10

(54) **COMPOSITION FOR TREATING FRAGILE X SYNDROME OR RELATED DEVELOPMENTAL DISORDERS, COMPRISING LISURIDE COMPOUND AS ACTIVE INGREDIENT**
ZUSAMMENSETZUNG ZUR BEHANDLUNG DES FRAGILEN X-SYNDROMS ODER VERWANDTER ENTWICKLUNGSSTÖRUNGEN MIT EINER LISURIDVERBINDUNG ALS WIRKSTOFF
COMPOSITION DE TRAITEMENT DU SYNDROME DE L'X FRAGILE OU DES TROUBLES DU DÉVELOPPEMENT ASSOCIÉS, COMPRENANT UN COMPOSÉ DE LISURIDE EN TANT QUE PRINCIPE ACTIF

(30) Priority: 08.01.2020 KR 20200002576
(43) Date of publication of application: 16.11.2022
(73) Proprietor: Neuroventi, Gwangjin-gu, Seoul 05029 (KR)
(72) Inventor: JOO, Sohyun, Seoul 01619 (KR); SHIN, Chanyoung, Seoul 07987 (KR); JEON, Sejin, Seoul 07217 (KR); AHN, Taejin, Seoul 06267 (KR)
(74) Representative: J A Kemp LLP
(86) International application number: PCT/KR2021/000227
(87) International publication number: WO 2021/141426

(56) References cited:
- KR-A- 20160 078 956
- US-A1- 2014 058 108
- US-A1- 2019 336 488
- SHAFIQ SAMREEN ET AL: "Using antipsychotics for behavioral problems in children", EXPERT OPIN PHARMACOTHER, vol. 19, no. 13, 13 August 2018 (2018-08-13), London, UK, pages 1475 - 1488, XP093105595, ISSN: 1465-6566, DOI: 10.1080/14656566.2018.1509069
- MCDONALD RICHARD J, HOROWSKI REINHARD: "Lisuride in the treatment of parkinsonism", EUROPEAN NEUROLOGY , vol. 22, no. 4, 1 January 1983 (1983-01-01), pages 240 - 255, XP009528955, ISSN: 0014-3022, DOI: 10.1159/000115567
- YAMASUE, HIDENORI; ARAN, ADI; BERRY-KRAVIS, ELIZABETH: "Emerging pharmacologic treatment options for fragile X syndrome", CURRENT OPINION IN NEUROLOGY, vol. 32, 2019, pages 635 - 640, XP009528954, ISSN: 1350-7540, DOI: 10.1097/WCO.0000000000000703

## Description

### [Technical field]

The present invention relates to a pharmaceutical composition for use in prevention or treatment of autism spectrum disorder, fragile X syndrome or a fragile X syndrome- related developmental disorder, and a health functional food composition for use in prevention or improvement of autism spectrum disorder, fragile X syndrome (FXS) or a fragile X syndrome-related developmental disorder.

### [Background Art]

Fragile X syndrome (FXS) is caused by a decrease in the Fmrl (fragile x mental retardation 1) gene functioning due to the amplification of the number of repeats of the CGG triple base at the 27.3 chromosomal location of the X gene. It also causes a lack of FMRP (Fragile X mental retardation protein), a protein produced through encoding the above gene. Considering that it is a disease caused by mutations in the X chromosome, the prevalence is about 1.5 times higher in men than in women. Fragile X syndrome has various comorbidities, and these diseases are mainly related to cranial nerve diseases. About 50% of patients have Attention Deficit Hyperactivity Disorder (ADHD) or autism, and there are also many patients with mental retardation, impulsivity, or language and cognitive disorders.

For research on the treatment of fragile X syndrome, mice lacking the Fmrl gene, a causative gene, were invented in 1994 and are still widely used. This animal model has phenotypes in several behavioral aspects, such as hyperactivity, lack of sociability, repetitive behavior, anxiety disorder, and cognitive decline depending on the strain of the mouse being used.

Most of the drugs used for the treatment of fragile X syndrome target group 1 metabolic-oriented glutamate receptors or inducible amino acid receptors to correct the imbalance of glutamate and glutamate receptors. In addition, they also target several proteins such as MMP9, MAP1B, PSD95, CaMKII, Arc, and STEP, which are involved in the FMRP protein production. However, difficulties in showing a therapeutic effect for just one symptom or failing in clinical trials have been reported.

On the other hand, Autism Spectrum Disorder (ASD) refers to a developmental disorder whose main symptoms include limitation of the range of behavioral interests, impairment of verbal and non-verbal communication, or decreased ability to understand social interaction. Previously, it was called autism, but recently, the diagnosis was revised to autism spectrum disorder, emphasizing that the degree and prognosis of autism are very diverse. ASD includes autism and pervasive developmental disorders (PDD), Asperger's Syndrome, Rett's Disorder, Childhood Disintegrative Disorder, and Pervasive Developmental Disorder (PDD NOS), not elsewhere classified. ASD is characterized by developmental delay or abnormal functioning before 3 years of age in at least one of the following areas: quality defects in social interaction and communication, hyperactivity, and restrictive/repetitive behaviors. In addition to the main symptoms, it is accompanied by symptoms of intellectual disability, sleep disturbances, gastrointestinal problems, epilepsy, or impulsive behaviors. However, the physiological cause and mechanism of the pathology of autism are not yet known, and the method for diagnosing ASD through molecular biological or pathological indicators has not yet been established. Thus, no drug is yet available to treat the cause of ASD, and only symptomatic drug treatment is given for accompanying symptoms such as epilepsy, self-harm, aggressive behavior, anxiety, or emotional disorder. Moreover, no drug is currently available that treats the core symptoms of ASD which are social deficits, and repetitive behaviors. Currently, research on the treatment response to existing psychiatric drugs such as fluoxetine and clozapine is being conducted at a rudimentary level. Experimental drugs such as D-cycloserine, oxytocin, Methallothionein I/II, and Gold are also utilized, but systematic efficacy studies have not been reported.

KR20160078956A discloses the use of metadoxine in the treatment of fragile X syndrome.

Efforts were being made to develop a therapeutic agent that can obtain a direct therapeutic effect for various symptoms of autism spectrum disorder and fragile X syndrome. Accordingly, the present invention was completed by confirming that the Fmrl-deficient fragile X syndrome animal model and the Cntnap2 deficient autistic animal model were symptomatically alleviated in terms of sociability and pre-pulse inhibition by the administration of lisuride.

### [Disclosure]

### [Technical Problem]

In the present invention, while efforts were being made to develop a therapeutic agent that can obtain a direct therapeutic effect for various symptoms of autism spectrum disorder and fragile X syndrome, the present invention was completed by confirming that an improvement effect on symptoms of lack of sociability and an improvement effect on pre-pulse suppression was proved in an Fmrl deficient fragile X syndrome animal model and Cntnap2 deficient autistic animal by administration of lisuride.

### [Summary of the Invention]

The present invention provides a pharmaceutical composition for use in prevention or treatment of autism spectrum disorder, fragile X syndrome (FXS) or a fragile X syndrome-related developmental disorder, comprising lisuride or a pharmaceutically acceptable salt thereof.

Lisuride may be a compound represented by the following formula (1):

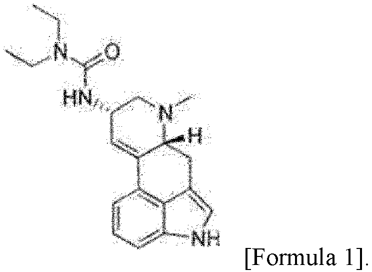

Autism spectrum disorder may be accompanied by one or more symptoms ranging from hyperactivity, repetitive behaviors, or lack of social skills.

Fragile X syndrome may be accompanied by one or more symptoms consisting of repetitive behavior, hyperactivity, reduced learning ability, lack of social skills, impulsivity, and anxiety symptoms.

The fragile X syndrome-related developmental disorders may include any one or more disorders from the group consisting of attention deficit hyperactivity disorder (ADHD), autism spectrum disorder (ASD), intellectual disability, cognitive impairment, impulse control disorders, and anxiety disorders.

The invention also provides a health functional food composition for use in prevention or improvement of autism spectrum disorder, fragile X syndrome (FXS) or a fragile X syndrome-related developmental disorder, comprising lisuride, a metabolite thereof or a pharmaceutically acceptable salt thereof.

### [Advantageous Effects]

The composition described herein has an effect of improving social behaviors and pre-pulse suppression in Fmrl-deficient mice, an animal model of fragile X syndrome, and in Cntnap2-deficient mice, an animal model of autism. Thus, the present composition can be effectively used as a preventive or therapeutic agent for autistic spectrum disorder or fragile X syndrome (FXS), or fragility X syndrome-related developmental disorders. In addition, the present invention can provide beneficial effects in a single drug compared to administering various drugs to alleviate several symptoms. In addition, the side effects caused by the administration of many drugs can be avoided and the economic burden can be reduced. Furthermore, since autism spectrum disorder or fragile X syndrome has homology with various mental developmental diseases such as cognitive impairment, attention deficit hyperactivity disorder, and impulsivity, the present composition can be used in the treatment of various neurodevelopmental disorders.

### [Description of Drawings]

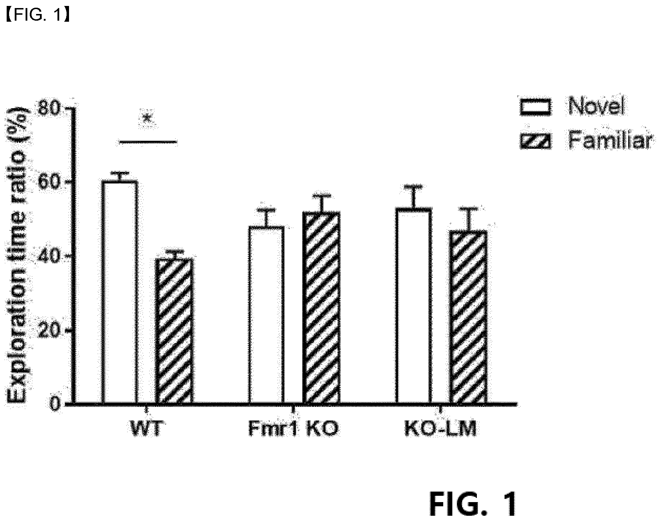

Fig. 1 is an analysis of the social improvement effect of lisuride in an animal model of fragile X syndrome (an animal model lacking Fmr1). It is judged to be social when spending more time with a stranger mouse over an empty compartment. (WT: wild-type mice (normal control), Fmr1 KO: knock-out mice deficient in FMRP expression, KO-LM: Fmrl-knockout mice administered with lisuride; * is p<0.05 vs WT controls).

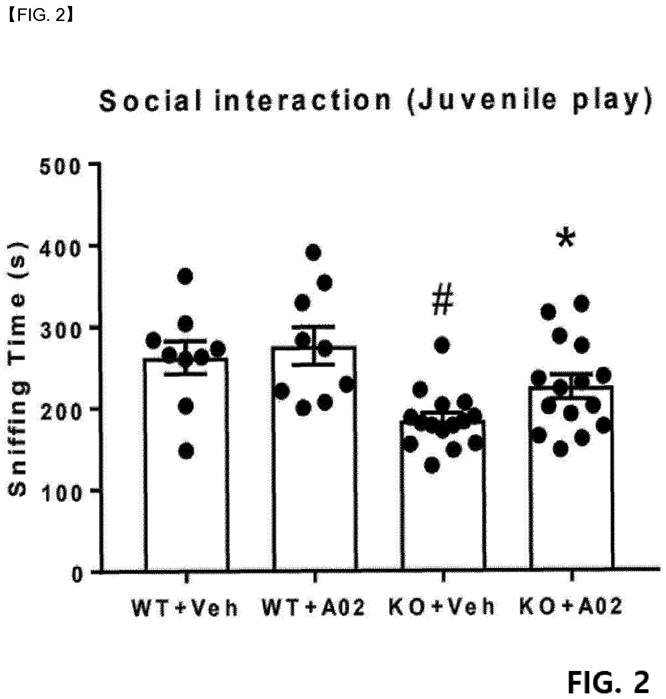

Fig. 2 shows the results of analyzing the social improvement effect of lisuride by performing a home-cage test in a fragile X syndrome animal model (Fmr1-knockout animal model). (WT+veh: wild-type mouse (normal control), WT+A02: wild-type mice administered with lisuride, KO+veh: Fmrl-knock-out mice, KO+A02: Fmr-1-knockout mice administered with lisuride; # is p<0.05 vs WT controls ; * is p<0.05 vs knock-out mice).

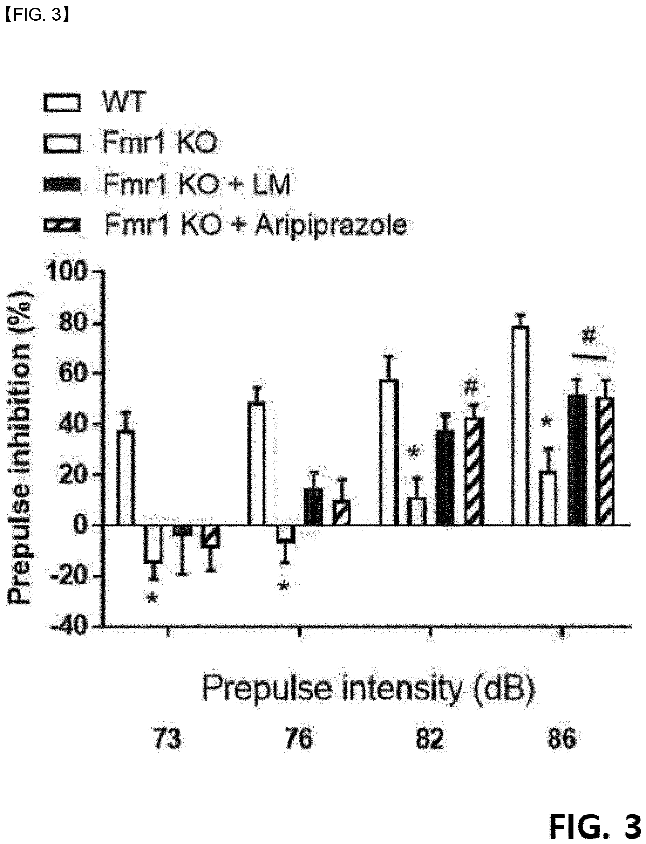

Fig. 3 shows the results of performing a pre-wave suppression test in a fragile X syndrome animal model (Fmr1-deficient animal model). (WT: wild-type mice (normal control), Fmr1 KO: knock-out mice deficient in FMRP expression, Fmrl KO+LM: Fmr1-knockout mice group administered with lisuride, Fmrl KO+Aripiprazol: Fmrl-knockout mice group administered with aripiprazole; * is p<0.05 vs WT controls; # is p<0.05 vs knock-out mice.

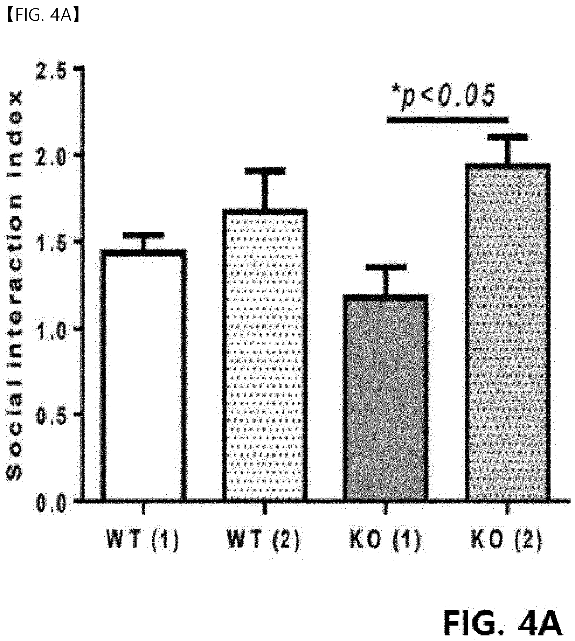

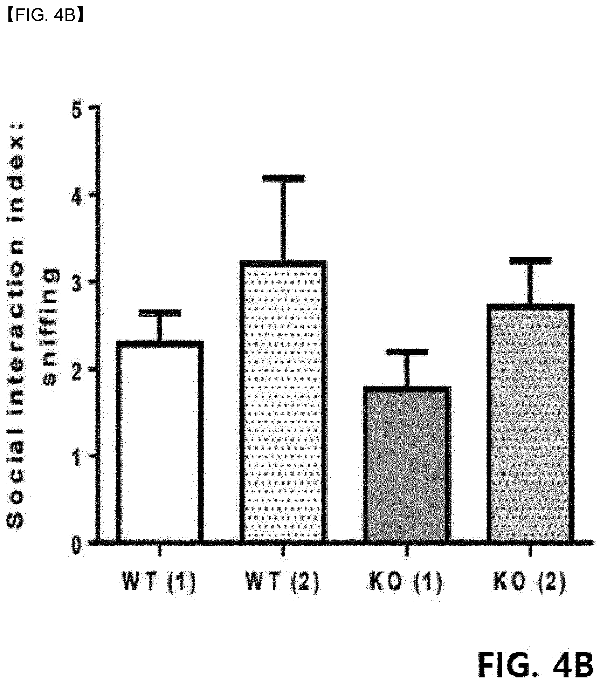

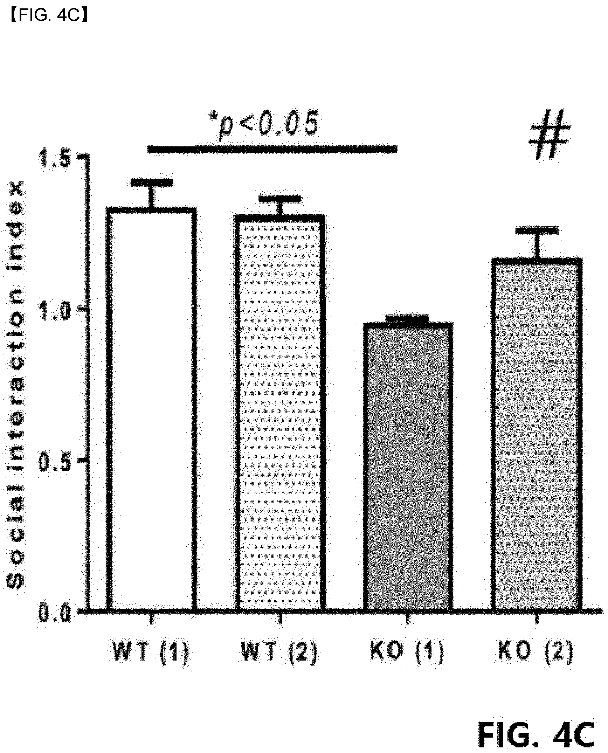

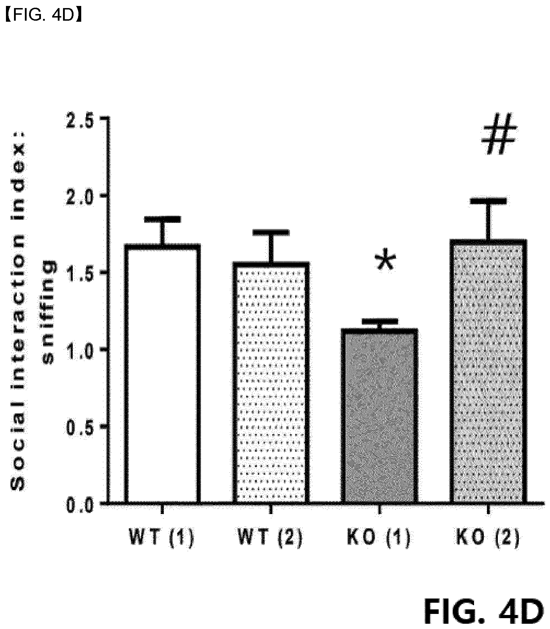

Fig. 4 is an analysis of the social improvement effect of lisuride in an autism animal model (Cntnap2-deficient animal model). Fig. 4A and 4B show the results of a single (1 treatment for 1 day) lisuride treatment, and Fig. 4C and 4D show the results of 5 days of treatment with lisuride (1 treatment per day). (WT(1): wild-type mice, WT(2): wild-type mice administered with lisuride, KO(1): knock-out mice deficient in FMRP expression, KO(2): Fmr1-knockout mice group administered with lisuride; * is p<0.05 vs WT controls; # is p<0.05 vs knock-out mice.

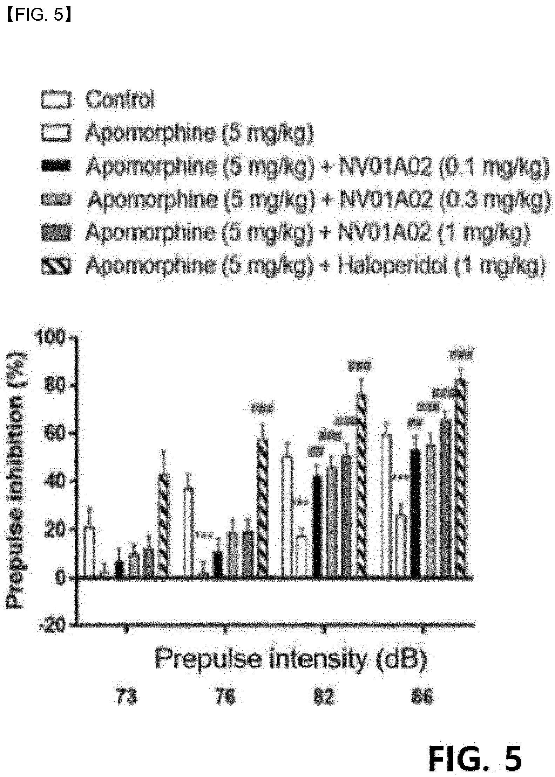

Fig. 5 is an analysis of the improvement effect of pre-wave suppression of lisuride (NV01A02) in an animal model treated with apomorphine in wild-type mice. *** is p<0.001 vs WT controls; # is p<0.05, ## is p<0.01, and ### is p<0.001 vs apomorphine-treated group.

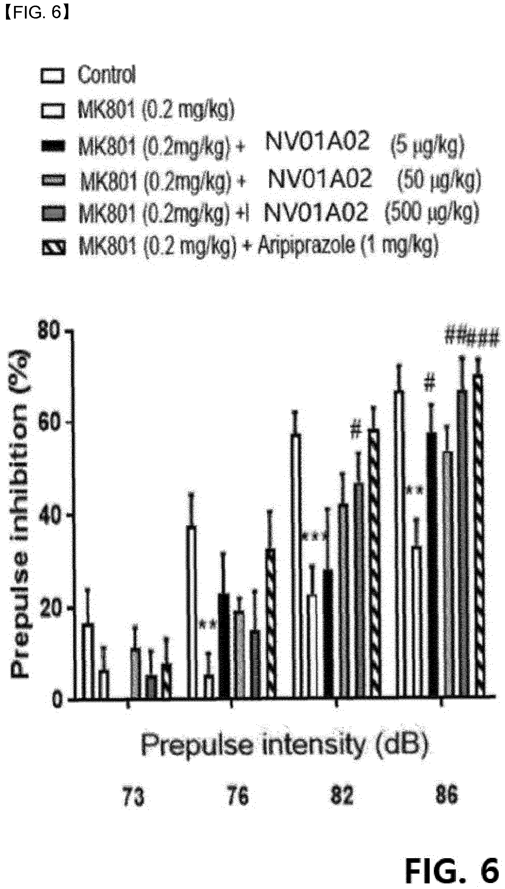

Fig. 6 is an analysis of the improvement effect of pre-pulse suppression of lisuride (NV01A02) in an animal model treated with MK-801 in wild-type mice. ** is p<0.01 and *** is p<0.001 vs WT controls; # is p<0.05, ## is p<0.01, and ### is p<0.001 vs MK801 treated group.

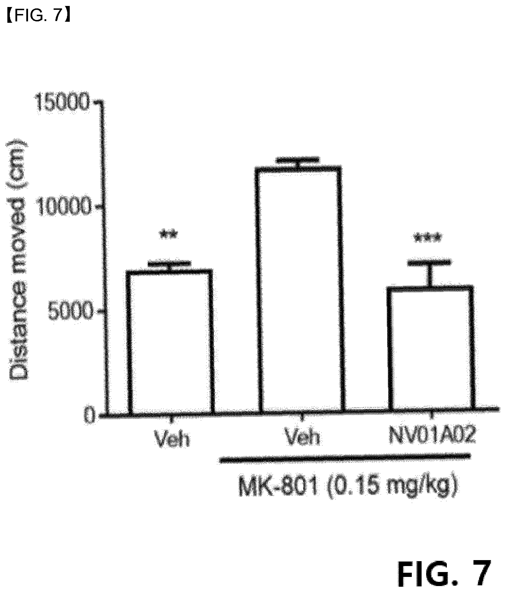

Fig. 7 is the results of analyzing the hyperactivity improvement effect of lisuride in an animal model treated with MK-801. (veh: Wild type mouse, NV01A02: lisuride treatment group; ** is p<0.001, *** is p<0.0001).

### [Mode of the Invention]

As described above, fragile X syndrome is characterized by cognitive deficits in learning and memory, lack of social skills, communication difficulties, and hyperactivity; and can be accompanied by autism and attention deficit hyperactivity disorder, which are also developmental disorders. Autism spectrum disorder is accompanied by symptoms such as hyperactivity, repetitive behaviors, or lack of social skills. However, drugs that can effectively and simultaneously treat one or more symptoms of autism spectrum disorder, fragile X syndrome, and any related developmental disorders, have not yet been reported.

Lisuride has therapeutic effects on direct or indirect symptoms of autism spectrum disorder, fragile X syndrome, or related developmental disorders (lack of sociability, prior wave suppression, etc.). A pharmaceutically acceptable salt thereof may be used as an active ingredient in a pharmaceutical composition for preventing and treating autism spectrum disorder, fragile X syndrome, and/or related developmental disorders.

Thus, described herein is a composition containing lisuride or a pharmaceutically acceptable salt thereof as an active ingredient, for preventing, improving, or treating symptoms of autism spectrum disorder, fragile X syndrome (FXS), or related developmental disorders.

Lisuride, an active ingredient of the composition for treating autism spectrum disorder, fragile X syndrome, or related developmental disorders described herein, is a compound represented by the following chemical formula 1. The molecular formula is C₂₀H₂₆N₄O and the molecular weight is 338.447 g/ mol. The IUPAC name of lisuride is 1,1-Diethyl-3-(7-methyl-4,6,6a,7,8,9 -hexahydro-indolo[4,3-fg]quinolin-9-yl)-urea:

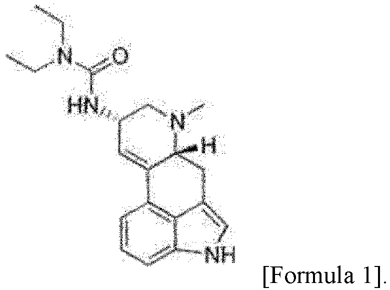

The term " pharmaceutically acceptable salt" or "salt thereof" may be an acid addition salt formed with a free acid. Acid addition salts can be prepared by conventional methods, for example, by dissolving the compound in an aqueous solution of excess acid and precipitating the salt with a water-miscible organic solvent such as methanol, ethanol, acetone, or acetonitrile. In addition, equimolar amounts of the compound and acid or alcohol in water (e.g., glycol monomethyl ether) may be heated and then the mixture may be evaporated to dryness, or the precipitated salt may be filtered off with suction. An inorganic acid or an organic acid may be used as the free acid. Non-limiting examples of the inorganic acid include hydrochloric acid, phosphoric acid, sulfuric acid, nitric acid, tartaric acid, and the like, and these may be used alone or in a combination of two or more. The "pharmaceutically acceptable salt" or "salt thereof" may be an acid addition salt formed with a free acid. Acid addition salts can be prepared by conventional methods, for example, by dissolving the compound in an aqueous solution of an excess of acid and precipitating the salt with a water-miscible organic solvent such as methanol, ethanol, acetone, or acetonitrile. In addition, equimolar amounts of the compound and acid or alcohol in water (e.g., glycol monomethyl ether) may be heated and then the mixture may be evaporated to dryness, or the precipitated salt may be filtered off with suction.

The salts of lisuride may include all salts of acidic or basic groups that may be present in the compounds of lisuride unless otherwise indicated. For example, the salt of lisuride may include sodium, calcium, and potassium salts of a hydroxyl group, and other cosmetically acceptable salts of an amino group include hydrobromide, sulfuric acid, hydrogen sulfate, phosphate, and hydrogen phosphate., dihydrogen phosphate, acetate, succinate, citrate, tartrate, lactate, mandelate, methanesulfonate (mesylate) and p- toluenesulfonate (tosylate) salts, and it can be prepared through a salt preparation method known in the art.

As described herein, "prevention" means any action that suppresses the onset or delays the onset by administration of the composition. As described herein, "improvement" or "treatment" refers to any action in which the symptoms of the disease are improved or beneficially changed by the administration of the composition, which preferably includes any one or more symptoms selected from the group consisting of hyperactivity symptoms and social deficit symptoms, but is not limited thereto, and the symptoms reported as symptoms of autism spectrum disorder.

As described herein, "autism spectrum disorder" preferably includes any one or more symptoms selected from the group consisting of hyperactivity, repetitive behaviors, and social deficit symptoms, but is not limited thereto, and can contain any symptoms reported as symptoms of autism spectrum disorder.

As described herein, "fragile X syndrome" preferably includes any one or more symptoms selected from the group consisting of repetitive behavior, hyperactivity, reduced learning ability, lack of sociability, impulsivity, and anxiety symptoms, but is not limited thereto, and may include any symptoms reported as symptoms of fragile X syndrome.

As described herein, "fragile X syndrome-related developmental disorder" preferably includes any one or more developmental disorders selected from the group consisting of attention deficit hyperactivity disorder (ADHD), autism spectrum disorder (ASD), intellectual disability, cognitive impairment, impulse control disorder, and anxiety disorder, but not limited thereto, and may include anything that has been reported as a concomitant disorder or disease of fragile X syndrome.

As described herein, the autism spectrum disorder may include autistic disorder, Rett's disorder, Asperger's syndrome, and Pervasive developmental disorder-not otherwise specified (PDD-NOS).

As described herein, "fragile X syndrome-related developmental disorder" preferably includes any one or more symptoms selected from the group consisting of repetitive behavior, hyperactivity, reduced learning ability, lack of social skills, impulsivity, and anxiety symptoms, but not limited to, and may include any symptoms reported as symptoms of "disorders associated with fragility X".

The present inventors confirmed the degree of social improvement according to the presence or absence of administration of lisuride in the fragile X syndrome animal model (Fmrl-deficient animal model) and autistic animal model (Cntnap2-deficient animal model), In an animal model deficient in Fmrl, sociability was decreased, but in mice treated with lisuride, sociability was improved. In addition, it was confirmed that the pre-pulse suppression was reduced in the Fmr1-deficient animal model, but recovered again by the lisuride treatment (Figs. 1 to 4).

The pharmaceutical preparation containing lisuride or a pharmaceutically acceptable salt thereof as an active ingredient may be formulated and administered in various oral or parenteral dosage forms at the time of clinical administration but is not limited thereto.

The oral administration may include, for example, tablets, pills, hard/soft capsules, solutions, suspensions, emulsifiers, syrups, granules, elixirs, and the like. rose, sucrose, mannitol, sorbitol, cellulose, and/or glycine), lubricants (e.g., silica, talc, stearic acid, and its magnesium or calcium salts and/or polyethylene glycol). Tablets may also contain binders such as magnesium aluminum silicate, starch paste, gelatin, methylcellulose, sodium carboxymethylcellulose, and/or polyvinylpyrrolidone, optionally starch, agar, alginic acid, or its sodium salt. It may contain disintegrants or effervescent mixtures and/or absorbents, colorants, flavoring agents, and sweetening agents.

The present pharmaceutical composition containing lisuride or a pharmaceutically acceptable salt thereof as an active ingredient, can be administered parenterally, and parenteral administration is by subcutaneous injection, intravenous injection, intramuscular injection, or intrathoracic injection. At this time, to formulate a dosage form for parenteral administration, a pharmaceutical composition containing lisuride or a pharmaceutically acceptable salt thereof as an active ingredient is mixed with water together with a stabilizer or buffer to prepare a solution or suspension, and it can be manufactured as an ampoule or vial unit dosage form. The composition may be sterilized and/or contain adjuvants such as preservatives, stabilizers, wetting agents or emulsification accelerators, salts and/or buffers for regulating osmotic pressure, and other therapeutically useful substances. It can be formulated according to the method of formulation or coating conventionally.

In the present composition, the dose of lisuride or a salt thereof to the human body may vary depending on the patient's age, weight, sex, dosage form, health status, and disease level. Based on an adult patient weighing 60 kg, generally, 0.001 to 1,000 mg/day, preferably 0.01 to 500 mg/day, may be administered in divided doses once or several times a day at regular time intervals according to the judgment of a doctor or pharmacist.

The composition described herein may contain the active ingredient in an amount of 0.1 to 90% by weight based on the total weight of the composition. However, the content is not necessarily limited thereto and may vary depending on the patient's condition, the type of disease, and the degree of progression.

Also described herein is a health functional food composition for preventing or improving symptoms of autism spectrum disorder, fragile X syndrome (FXS), or related disorders containing lisuride or a salt thereof as an active ingredient. The detailed contents of lisuride are the same as described above.

As described herein, the term "health functional food" refers to food manufactured and processed in the form of tablets, capsules, powders, granules, liquids, pills, etc. using raw materials or ingredients useful for the human body. Here, 'functionality' refers to obtaining useful effects for health purposes, such as regulating nutrients or physiological effects on the structure and function of the human body. The health functional food described hereincan be prepared by a method commonly used in the art, and during the manufacture, it can be prepared by adding raw materials and components commonly added in the art. In addition, the dosage form of the health functional food may be manufactured without limitation as long as it is a dosage form recognized as a health functional food. The health functional food composition described herein has the advantage that there are no side effects that may occur during long-term administration using food as raw material, unlike general drugs, and has excellent portability, thereby it can be taken as a supplement to enhance the effect of alleviating the symptoms of fragile X syndrome or its related developmental disorders.

In the present health functional food for the prevention or improvement of autism spectrum disorder, fragile X syndrome, or related developmental disorders, when lisuride is used as an additive to a health functional food, it is added as it is or mixed with other food or food ingredients. They can be used together and can be used appropriately according to a conventional method. The mixing amount of the active ingredient may be appropriately determined according to each purpose of use, such as prevention, health, or treatment.

The formulation of health functional food may be in the form of powder, granules, pills, tablets, and capsules, as well as in the form of general food or beverages.

The type of food is not particularly limited, and examples of food to which the substance can be added include meat, sausage, bread, chocolate, candy, snacks, confectionery, pizza, ramen, other noodles, gum, and dairy products including ice cream, various soups, beverages, teas, drinks, alcoholic beverages, vitamin complexes, etc., and may include all foods in a conventional sense.

The mixing amount of the active ingredient may be appropriately determined depending on the purpose of its use (for prevention or improvement). **In** general, the amount of the compound (active ingredient) in the health food may be added in an amount of 0.1 to 90% by weight based on the total weight of the food. However, in the case of long-term intake for health and hygiene or health control, the amount may be less than the above range, and since there is no problem in terms of safety, the active ingredient may be used in an amount above the above range.

Beverages among functional foods may contain various flavoring agents or natural carbohydrates as additional ingredients like conventional beverages. The above-mentioned natural carbohydrates may be monosaccharides such as glucose and fructose, disaccharides such as maltose and sucrose, polysaccharides such as dextrin and cyclodextrin, and sugar alcohols such as xylitol, sorbitol, and erythritol. As for the sweetener, natural sweeteners such as taumatine and stevia extract, and synthetic sweeteners such as saccharin and aspartame may be used. The ratio of the natural carbohydrate may be about 0.01 to 0.04 g, preferably about 0.02 to 0.03 g per 100 mL of the beverage.

In addition to the above, the health functional food for the prevention or improvement of fragile X syndrome or related developmental disorders may include various nutrients, vitamins, electrolytes, flavoring agents, coloring agents, pectic acid and its salts, alginic acid, and its salts, organic acids, and protective properties. It may contain colloidal thickeners, pH adjusters, stabilizers, preservatives, glycerin, alcohols, and carbonating agents used in carbonated beverages. In addition, the health functional food composition for preventing or improving fragile X syndrome or related developmental disorders described herein may contain fruit for the production of natural fruit juice, fruit juice beverage, and vegetable beverage. These components may be used independently or in combination. The ratio of these additives is not limited but is generally selected in the range of 0.01 to 0.1 parts by weight relative to 100 parts by weight of the functional food described herein.

Hereinafter, the present invention will be described in more detail through examples. These examples are only for illustrating the present invention.

### Example 1: Confirmation of the social improvement effect and the pre-pulse suppression improvement effect of Lisuride in the fragile X syndrome animal model

### Preparation of laboratory animals and drugs

Purchased a 5-week-old Fmrl knockout mouse (Jackson Laboratory, USA) weighing about 22 g to 24 g, and acclimatized to an environment with a temperature of 23±2°C and humidity of 55±10% and a light-dark cycle of 12 hours while allowing them to freely consume water and feed (Experimental Animal Center, Konkuk University). As experimental animals, wild-type (WT) with normal FMRP expression and male KO mice lacking FMRP (fragile X mental retardation protein) expression (fragile X syndrome; FXS) were used. Dosing and experiments were performed according to the age of the week. Dosing was performed at the same time. Behavioral experiments were conducted 30 minutes after administration. It was divided into groups administered with 1 mg/kg each of lisuride (Sigma Aldrich) in FMRP expression-deficient knock-out (Knock-Out) mice, wild-type (Wild Type) mice, and 0.25 (v/v) % DMSO in FMRP expression-deficient knock-out mice (6 mice per group). For the drug administration group, lisuride was dissolved in 0.25 (v/v) % DMSO and then administered intraperitoneally at a dose of 50 ug/kg. Meanwhile, 0.25 (v/v) % DMSO was intraperitoneally administered to the control group.

### Confirmation of Social Improvement Effect of Lisuride in Fragile X Syndrome Animal Model 1

To confirm the therapeutic effect of lisuride on social deficits, one of the phenotypes of fragile X syndrome and related developmental disorders, a three-chamber test was performed in the Fmrl-deficient animal model. If the animal model spent more time with a novel mouse rather than a familiar mouse, it is judged to be social. The test animal was exposed to the stimulus mouse for 10 minutes, and to a new stimulus mouse (novel) for another 10 minutes. In this case, the old mouse is called familiar and the new mouse is called a novel mouse. The time the test mouse explored each stimulus mouse was measured and expressed as %. As a result of the experiment, it was confirmed that the wild type spent more time with the novel mouse than the familiar mouse, indicating normal sociability. On the other hand, the mice deficient in Fmrl spent more time with the familiar mouse than the novel mouse, indicating impaired sociability. When Fmrl-deficient mice were treated with lisuride, more time was spent with the novel mouse than with the familiar mouse, indicating that sociability was improved through the treatment with lisuride (Fig. 1).

### Confirmation of Social Improvement Effect of Lisuride in Fragile X Syndrome Animal Model 2

To confirm the therapeutic effect of lisuride on social deficits, one of the phenotypes of developmental disorders, a home-cage test was performed using an Fmr1-deficient animal model. A home-cage test evaluates sociability in the breeding cage removing the possible confounders of stress due to abrupt changes in the environment. After raising the group of animals to be tested together, a wire cage was placed inside the home cage and allowed the animals to acclimatize for 30 minutes. A stimulus mouse was placed in a wire cage and the duration of sniffing by the subject mice to the wire cage was measured for 15 minutes. Lisuride was administered once daily for 5 days. The sniffing time was reduced in Fmr1-deficient mice, but it was confirmed that the sniffing time increased again when lisuride was administered, suggesting that sociability was improved by lisuride treatment (Fig. 2).

### Confirmation of improvement effect of pre-pulse suppression of Lisuride in Fragile X Syndrome animal model

The startle response was confirmed using an Fmr1-deficient animal model. Mice were divided into groups, and 0.9% physiological saline (saline solution) or lisuride was intraperitoneally administered to wild-type or knock-out mice. Thereafter, sensorimotor gating was evaluated through a pre-wave suppression test (converted using Equation 1 below). As a result of the experiment, it was confirmed that the pre-pulse suppression was reduced in Fmrl-deficient mice, but the pre-pulse suppression was restored when lisuride was treated (Fig. 3). Pre-pulse suppression (%) = 100 - [(startle response to pre-stimulus + stimulus)/(start- up response to stimulus)] * 100

### Example 2: Confirmation of the effect of improving sociability and improvement of pre-pulse suppression of Lisuride in an autism animal model

### Preparation of laboratory animals and drugs

Purchased a 5-week-old Cntnap2 knockout mouse (Jackson Laboratory, USA) weighing about 22 g to 24 g and acclimatize to an environment with a temperature of 23 ± 2°C, humidity of 55 ± 10%, and a light-dark cycle of 12 hours while allowing them to freely consume water and feed. After breeding (Konkuk University Laboratory Animal Center), they were used for experiments. Wild-type (WT) mice showing normal expression of Cntnap2 and KO mice lacking Cntnap2 expression were used. The dosing and testing were performed according to the age of the week. Dosing was performed at the same time. Behavioral experiments were conducted 30 minutes after administration. It was divided into groups administered with 1 mg/kg each of lisuride (Sigma Aldrich) or 0.25 (v/v) % DMSO in Cntnap2 expression-deficient knock-out mice or wild-type mice. For the drug administration group, lisuride was dissolved in 0.25% DMSO and then administered intraperitoneally at a dose of 50 ug /kg. Meanwhile, 0.25 (v/v) % DMSO was intraperitoneally administered to the control group.

### Confirmation of social improvement effect of Lisuride in autism animal model (Cntnap2-deficient animal model)

To confirm the therapeutic effect of Iisuride on the lack of sociability, one of the phenotypes of developmental disorders, a three-chamber test was performed using an animal model lacking Cntnap2. Experimental results were expressed as a social interaction index by measuring the time the test mouse approaches or sniffs each stimulus mouse. The higher the social interaction index means more time spent or sniffing in the novel over the familiar mouse.

Cntnap2 deficient mice spent more time or sniffed more with the familiar mice than novel mice. The KO group's social interaction index was decreased compared to wild-type mice. On the other hand, when Cntnap2-deficient mice were treated with lisuride, they spent more time and sniffed more with the novel mouse than the familiar mouse (FIG. 4).

### Example 3: Confirmation of the improvement effect of pre-pulse suppression and hyperactivity improvement of lisuride in an animal model treated with apomorphine or MK-801

### Confirmation of improvement effect of pre-wave suppression of Lisuride

Startle response was confirmed in an animal model treated with apomorphine or MK-801 in wild-type mice. Mice were divided into groups, and 0.9 (w/v)% of physiological saline (saline solution) or lisuride was administered intraperitoneally to each group. Thereafter, sensorimotor gating was evaluated through a pre-wave suppression test (converted using Equation 1 above). As a result of the experiment, it was confirmed that, when wild-type mice were treated with apomorphine or MK-801, the pre-pulse suppression was reduced, but when lisuride was treated after apomorphine or MK-801, it was confirmed that the pre-pulse suppression was restored (Fig. 5, Fig. 6).

### Confirmation of effect of Lisuride in improving hyperactivity

To confirm the hyperactivity improvement effect of lisuride, an open-field test was performed using an animal model in which MK-801 was administered to wild-type mice, and the results are shown in Fig. 7. As a result of the experiment, hyperactivity was observed when MK-801 was administered but was significantly reduced when treated with lisuride.

Hereinafter, an example of the preparation of a pharmaceutical or food containing lisuride as an active ingredient will be described, not intended to limit the invention but merely to describe it in detail. Using the active ingredient, the pharmaceutical or food composition of Preparation Example 1 or 2 was prepared according to a conventional method according to the following compositional components and composition ratios.

### [Preparation Example 1] Preparation of pharmaceutical composition

<1-1> Preparation of powder
Lisuride 20 mg
Lactose hydrate 100 mg
Talc 10 mg
The above ingredients were mixed and filled in an airtight bag to prepare a powder.

**<1-2> Preparation of tablets**
Lisuride 10 mg
Corn Starch 100 mg
Lactose hydrate 100 mg
Magnesium stearate 2 mg
After mixing the above ingredients, tablets were prepared by tableting according to a conventional manufacturing method of tablets.

**<1-3> Preparation of capsules**
Lisuride 10 mg
Microcrystalline Cellulose 3 mg
Lactose hydrate 14.8 mg
Magnesium stearate 0.2 mg
After mixing the above ingredients, the capsules were prepared by filling in gelatin capsules according to a conventional manufacturing method of capsules.

**<1-4> Preparation of injection**
Lisuride 10 mg
Mannitol 180 mg
Sterile distilled water for injection 2974 mg
Sodium monohydrogen phosphate 26 mg
After mixing the above ingredients, the content of the above components per 1 ampoule (2 mL) was prepared according to a conventional method for preparing injections.

**<1-5> Preparation of liquid preparation**

| | |
|---|---|
| Lisuride | 10 mg |
| Isomerized sugar | 10 mg |
| Mannitol | 5 mg |
| Purified water | appropriate amount |
| Lemon flavor | appropriate amount |

The above ingredients were dissolved by adding each ingredient to purified water according to a conventional manufacturing method. After adding an appropriate amount of lemon flavor, purified water was added to adjust the total volume to 100 mL, sterilized, and filled in a brown bottle to prepare a solution.

### [Production Example 2] Preparation of health functional food

### <2-1> Manufacturing of health supplements

Lisuride 10 mg
Vitamin A acetate 70 µg
Vitamin E 1.0 mg
Vitamin B₁ 0.13 mg
Vitamin B₂ 0.15 mg
Vitamin B₆ 0.5 mg
Vitamin B₁₂ 0.2 µg
Vitamin C 10 mg
Biotin 10 µg
Nicotinamide 1.7 mg
Folic acid 50 µg
Calcium pantothenate 0.5 mg
Mineral mixture appropriate amount
Ferrous sulfate 1.75 mg
Zinc oxide 0.82 mg
Magnesium carbonate 25.3 mg
Potassium monophosphate 15 mg
Dibasic calcium phosphate 55 mg
Potassium citrate 30 mg
Calcium carbonate 100 mg
Magnesium chloride 24.8 mg

The composition ratio of the vitamin and mineral mixture is relatively suitable for health food in a preferred embodiment, but the mixing ratio may be arbitrarily modified to prepare granules and can be used for preparing health food compositions according to a conventional method.

### <2-2 > Manufacturing of health drinks

Lisuride 10 mg
Vitamin C 15 g
Vitamin E (powder) 100 g
Iron lactate 19.75 g
Zinc oxide 3.5 g
Nicotinic acid amide 3.5 g
Vitamin A 0.2 g
Vitamin B1 0.25 g
Vitamin B2 0.3 g
Purified water appropriate amount

After mixing the above ingredients according to a conventional health drink manufacturing method, stirring and heating at 85°C. for about 1 hour, the resulting solution was filtered and obtained in a sterilized 2L container, sealed, sterilized, refrigerated, and then used in the manufacture of health beverage compositions.

Although the composition ratio is prepared by mixing ingredients suitable for relatively favorite beverages in a preferred embodiment, the mixing ratio may be arbitrarily modified according to regional and national preferences such as demand class, demanding country, and use.

## Claims

1. A pharmaceutical composition for use in prevention or treatment of autism spectrum disorder, fragile X syndrome (FXS) or a fragile X syndrome-related developmental disorder, comprising lisuride or a pharmaceutically acceptable salt thereof.

2. The pharmaceutical composition for use according to claim 1, wherein the lisuride is represented by the following formula (1):

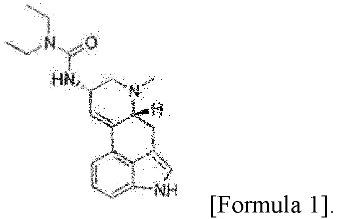

3. The pharmaceutical composition for use according to claim 1, wherein the autism spectrum disorder is accompanied by one or more symptoms selected from hyperactivity or social deficit.

4. The pharmaceutical composition for use according to claim 1, wherein the fragile X syndrome is accompanied by one or more symptoms selected from the group consisting of repetitive behavior, hyperactivity, reduced learning ability, lack of social skills, impulsivity and anxiety symptoms.

5. The pharmaceutical composition for use according to claim 1, wherein the fragile X syndrome-related developmental disorder includes one or more symptoms selected from the group consisting of attention deficit hyperactivity disorder (ADHD), autism spectrum disorder (ASD), intellectual disability, cognitive impairment, and impulse control disorder and anxiety disorder.

6. A health functional food composition for use in prevention or improvement of autism spectrum disorder, fragile X syndrome (FXS) or a fragile X syndrome-related developmental disorder, comprising lisuride or a pharmaceutically acceptable salt thereof.

## Patentansprüche

1. Pharmazeutische Zusammensetzung zur Verwendung bei der Prävention oder Behandlung von Autismus-Spektrum-Störung, Fragilem-X-Syndrom (FXS) oder einer mit Fragilem-X-Syndrom zusammenhängenden Entwicklungsstörung, umfassend Lisurid oder ein pharmazeutisch annehmbares Salz davon.

2. Pharmazeutische Zusammensetzung zur Verwendung gemäß Anspruch 1, wobei das Lisurid durch die folgende Formel (1) dargestellt ist:

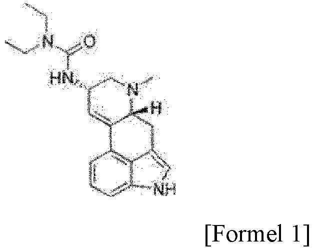

3. Pharmazeutische Zusammensetzung zur Verwendung gemäß Anspruch 1, wobei die Autismus-Spektrum-Störung von einem oder mehreren Symptomen begleitet wird, die ausgewählt sind aus Hyperaktivität oder sozialem Defizit.

4. Pharmazeutische Zusammensetzung zur Verwendung gemäß Anspruch 1, wobei das Fragile-X-Syndrom von einem oder mehreren Symptomen begleitet wird, die ausgewählt sind aus der Gruppe, bestehend aus repetitivem Verhalten, Hyperaktivität, verminderter Lernfähigkeit, Mangel an sozialen Fähigkeiten, Impulsivität und Angstsymptomen.

5. Pharmazeutische Zusammensetzung zur Verwendung gemäß Anspruch 1, wobei die mit dem Fragilen-X-Syndrom zusammenhängende Entwicklungsstörung ein oder mehrere Symptome beinhaltet, die ausgewählt sind aus der Gruppe, bestehend aus Aufmerksamkeitsdefizit-/Hyperaktivitätsstörung (ADHD), Autismus-Spektrum-Störung (ASD), geistiger Behinderung, kognitiver Beeinträchtigung und Impulskontrollstörung und Angststörung.

6. Funktionelle Gesundheitsnahrungszusammensetzung zur Verwendung bei der Prävention oder Verbesserung von Autismus-Spektrum-Störung, Fragilem-X-Syndrom (FXS) oder einer mit Fragilem-X-Syndrom zusammenhängenden Entwicklungsstörung, umfassend Lisurid oder ein pharmazeutisch annehmbares Salz davon.

## Revendications

1. Composition pharmaceutique pour utilisation dans la prévention ou le traitement de trouble du spectre de l'autisme, de syndrome de l'X fragile (FXS), ou d'un trouble développemental lié au syndrome de l'X fragile, comprenant du lisuride ou un sel pharmaceutiquement acceptable de celui-ci.

2. Composition pharmaceutique pour utilisation selon la revendication 1, dans laquelle le lisuride est représenté par la formule suivante (1) :

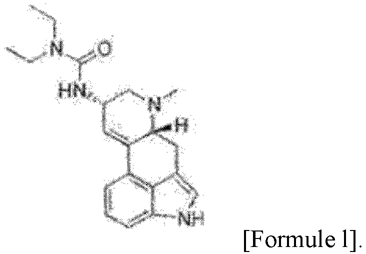

3. Composition pharmaceutique pour utilisation selon la revendication 1, dans laquelle le trouble du spectre de l'autisme est accompagné par un ou plusieurs symptômes sélectionnés parmi l'hyperactivité ou la déficience sociale.

4. Composition pharmaceutique pour utilisation selon la revendication 1, dans laquelle le syndrome de l'X fragile est accompagné par un ou plusieurs symptômes sélectionnés parmi le groupe constitué de symptômes de comportement répétitif, d'hyperactivité, de capacité d'apprentissage réduite, de manque d'aptitude sociale, d'impulsivité, et d'anxiété.

5. Composition pharmaceutique pour utilisation selon la revendication 1, dans laquelle le trouble développemental lié au syndrome de l'X fragile inclut un ou plusieurs symptômes sélectionnés parmi le groupe constitué de trouble déficitaire de l'attention avec hyperactivité (TDAH), de trouble du spectre de l'autisme (TSA), de déficience intellectuelle, de trouble cognitif, et de trouble de contrôle des impulsions, et de trouble anxieux.

6. Composition alimentaire fonctionnelle de santé pour utilisation dans la prévention ou l'amélioration de trouble du spectre de l'autisme, de syndrome de l'X fragile (FXS), ou de trouble développemental lié au syndrome de l'X fragile, comprenant du lisuride ou un sel pharmaceutiquement acceptable de celui-ci.
